# EUROPÄISCHE PATENTSCHRIFT

(11) **EP 3 437 451 B1**
(45) Veröffentlichungstag und Bekanntmachung des Hinweises auf die Patenterteilung: **23.12.2020**
(21) Anmeldenummer: 18185776.4
(22) Anmeldetag: 26.07.2018
(51) Int. Cl.: A01C 21/00, G01J 3/18, G01J 3/44, G01N 21/65, G01J 3/02, G01N 33/24, G01N 33/00, A01B 79/00

(54) **LANDWIRTSCHAFTLICHE MASCHINE MIT EINER SPEKTROMETERANORDNUNG**
AGRICULTURAL MACHINE WITH A SPECTROMETER ASSEMBLY
MACHINE AGRICOLE AVEC UN DISPOSITIF FORMANT SPECTROMÈTRE

(30) Priorität: 02.08.2017 DE 102017213419
(43) Veröffentlichungstag der Anmeldung: 06.02.2019
(73) Patentinhaber: Deere & Company, Moline, IL 61265 (US)
(72) Erfinder: Kormann, Georg, 68163 Mannheim (DE); Haiges, Wolfram, 68163 Mannheim (DE); Schade, Peter, 68163 Mannheim (DE); Correns, Nico, 68163 Mannheim (DE); Fischer, Bernhard, 68163 Mannheim (DE); Grunert, Fred, 68163 Mannheim (DE)
(74) Vertreter: Holst, Sönke

(56) Entgegenhaltungen:
- EP-A1- 3 186 603
- WO-A1-2008/090350
- WO-A1-2014/023810
- US-A- 5 038 040
- US-A1- 2016 349 167

## Beschreibung

Die Erfindung betrifft eine landwirtschaftliche Maschine mit einer Spektrometeranordnung zur Untersuchung einer Probe.

### Stand der Technik

Ein Bestreben der Präzisions-Landwirtschaft ist, mit gegebenen Mitteln ein möglichst gutes Ergebnis beim Anbau von Pflanzen zu erzielen. Man versucht daher, beim Säen, Düngen, Spritzen etc. die Arbeitsparameter, wie Sätiefe, Abstände, Saatgutart, Art des Düngemittels, Ausbringmengen etc. zu optimieren, basierend auf zuvor gewonnenen Daten, die vor dem Arbeitsvorgang oder während des Arbeitsvorgangs durch Sensoren oder andere Verfahren ermittelt werden und beispielsweise die Bodenart, Bodenfeuchte und Bodeninhaltsstoffe und/oder die Inhaltsstoffe auszubringender Stoffe (Dünger, Gülle) und/oder Eigenschaften von Pflanzen (Wachstumszustand, Inhaltsstoffe) betreffen. Neben chemischen Analysen zur Ermittlung von Inhaltsstoffen des Bodens, z.B. von Phosphor, Kalium oder organischen Inhaltsstoffen wurden sensorbasierte Vorgehensweisen beschrieben, die beispielsweise auf Gammaspektrometern und optischen Spektrometern basieren und bei der Überfahrt über ein Feld anhand von sensorisch erfassten Signalen und daraus abgeleiteten Informationen die Bodeninhaltsstoffe erfassen, um sie zu kartieren und/oder eine Vorrichtung zur Düngemittelausbringung darauf basierend anzusteuern (z.B. DE 10 2013 208 680 A1). Analog sind im Stand der Technik mit Nahinfrarotspektrometern ausgerüstete Sensoren zur Analyse der Inhaltsstoffe von Erntegut (EP 1 053 671 A1) und Gülle (NL 1015440 C) beschrieben worden, wobei die US 5 038 040 A eine Beaufschlagung der Probe mit einem kreisförmigen Lichtfleck und die Analyse des reflektierten Lichts mit einem dispersiven Monochromator auf eine Detektorreihe beschreibt, während die WO 2014/023810 A1 ein sogenannte passives Spektrometer beschreibt, bei dem die Probe durch Umgebungs- (Sonnen-) Licht beleuchtet und das reflektierte Licht durch einen Schlitz im Gehäuse des Spektrometers auf ein dispersives Element fällt, von dem es auf einen zweidimensionalen Bildsensor fällt. Manche mineralische Inhaltsstoffe (z.B. Phosphor und Kalium) von Böden und anderen Substanzen, wie Flüssigkeiten (Gülle), lassen sich jedoch durch NIR-Spektroskopie nur schlecht erfassen. Die als US 2016/0349167 A1 beschreibt eine Beleuchtung der Probe über einen konkaven Spiegel und eine Analyse des reflektierten Lichts mit einem dispersiven Monochromator und einem zweidimensionalen Detektor, jedoch unter Ausgabe nur eines einzigen Spektrums für die Probe. In der WO 2016/030674 A1 wird ein Raman-Spektrometer beschrieben, bei dem die Probe mit einem punkt- oder linienförmigen Lichtstrahl beleuchtet wird. Das von der Probe reflektierte Licht wird in zwei unterschiedliche Wellenlängenbereiche aufgeteilt und die beiden Wellenlängenbereiche werden einem dispersiven Element zugeführt, von dem das wellenlängig unterschiedlich abgelenkte Licht an zwei voneinander beabstandeten Stellen eines zweidimensionalen Detektors einläuft. Der zweidimensionale Detektor liefert demnach zwei verschiedene Spektren, die von derselben Stelle der Probe kommen.

Es wurde zudem vorgeschlagen, Inhaltsstoffe von Böden, insbesondere Phosphor, mit einem Raman-Spektrometer zu untersuchen (US 2007/0013908 A1; J. Kruse et al., Innovative methods in soil phosphorus research: A review, J. Plant Nutr. Soil Sci. 2015, 178, 43-88 und S. Luna et al., Classification of soil samples based on Raman spectroscopy and X-ray fluorescence spectrometry combined with chemometric methods and variable selection, Anal. Methods, 2014, 6, 8930-8939). Bei der Raman-Spektroskopie wird die Probe mit Licht bestrahlt und inelastische Lichtstreuung, die durch einen Energieübertrag der Anregungswellenlänge in Rotations- und Vibrationsschwingungen von Oberflächenmolekülen der Probe oder umgekehrt bewirkt wird, wird erfasst und analysiert. Die Wellenlängenverschiebung ist spezifisch für die Molekülsorte und kann zu deren Identifikation herangezogen werden.

### Aufgabe

Im Stand der Technik wird bei spektroskopischen Untersuchungen des Bodens oder einer anderen Probe jeweils eine einzige Stelle der Probe mit Licht beaufschlagt. Die Probe setzt sich in der Regel aus relativ kleinen Partikeln zusammen, deren Abmessungen im mm-Bereich oder darunter liegen. Wählt man den Durchmesser des Anregungslichtflecks kleiner als die Partikelgröße, erhält man wegen der Messfläche, die kleiner als die Partikelgröße ist, nicht unbedingt Ergebnisse, die für die gesamte Probe repräsentativ und korrekt wären. Es wäre demnach eine sehr große Anzahl an Messwerten aufzunehmen, um sinnvolle Ergebnisse zu erzielen, was die Messzeit unangemessen lang werden lässt. Alternativ könnte man den Anregungslichtfleck größer als die Partikelgröße wählen, was jedoch den Nachteil mit sich bringt, dass ggf. nicht-repräsentative Partikel (im Falle der Analyse einer Kornprobe z.B. Nichtkornbestandteile) mit analysiert werden, welche das Messergebnis verfälschen. Diese Problematik besteht bei jeder Art von dispersiver Spektroskopie zur Untersuchung von biologischen oder geologischen Proben, d.h. nicht nur bei der Raman-Spektroskopie, sondern auch bei optischer Spektroskopie im (nah-) infraroten oder sichtbaren Wellenlängenbereich, bei welcher die Probe mit breitbandigem Licht bestrahlt und das von der Probe reflektierte oder transmittierte Licht wellenlängenaufgelöst analysiert wird.

Die Erfindung hat sich zur Aufgabe gesetzt, die genannten Nachteile zu vermeiden oder zumindest zu vermindern.

### Lösung

Diese Aufgabe wird erfindungsgemäß durch die Lehre des Patentanspruchs 1 gelöst, wobei in den weiteren Patentansprüchen Merkmale aufgeführt sind, die die Lösung in vorteilhafter Weise weiterentwickeln.

Eine Spektrometeranordnung zur Untersuchung einer beliebigen Probe umfasst eine Quelle für elektromagnetische Wellen (z.B. eine oder mehrere Leuchtdiode(n) oder Laserdiode(n)), Übertragungsmittel zum Übertragen der Wellen von der Quelle zur Probe (z.B. eine Linse oder ein Glasfaserkabel), ein dispersives Element (z.B. ein Gitter oder ein Prisma), das mit von der Probe einlaufenden, reflektierten oder transmittierten Wellen beaufschlagbar ist und die einlaufenden Wellen wellenlängenabhängig in unterschiedliche räumliche Richtungen ablenkt, und einen Detektor mit mehreren Nachweiselementen zur Umsetzung der vom dispersiven Element einlaufenden Wellen in elektrische Signale. Die Übertragungsmittel sind ausgelegt, die Probe strichförmig mit den elektromagnetischen Wellen zu beleuchten und die Nachweiselemente des Detektors sind zweidimensional angeordnet, wobei die von unterschiedlichen Orten der strichförmig mit elektromagnetischen Wellen beaufschlagten Probe ausgehenden elektromagnetischen Wellen entlang einer ersten Richtung an unterschiedlichen Nachweiselementen des Detektors und aufgrund der durch das dispersive Element bewirkten, wellenlängenabhängigen Ablenkung entlang einer zweiten Richtung an unterschiedlichen Nachweiselementen des Detektors einlaufen. Die Probe wird somit entlang der strichförmigen Beleuchtung separat spektral analysiert, was die oben erwähnten Probleme vermeidet oder wenigstens mindert.

Mit anderen Worten werden die von der Quelle erzeugten, elektromagnetischen Wellen, bei denen es sich um sichtbares Licht oder im darüber oder darunter liegenden Frequenzbereich (UV, (N)IR oder Mikrowellen) liegende Wellen handeln kann, durch die Übertragungsmittel nicht - wie im Stand der Technik - mehr oder weniger kreis- oder punktförmig (sozusagen nulldimensional), sondern strichförmig (eindimensional) auf die Probe projiziert (abgebildet). Hierfür kann beispielsweise eine Zylinderlinse, ein Prisma und/oder ein sich hin- und her bewegendes oder rotierendes Ablenkungs- oder Leitelement für die elektromagnetischen Wellen, z.B. ein Spiegel, verwendet werden. Die Abmessung des beleuchteten Bereichs der Probe quer zur Längsrichtung des Striches kann möglichst klein gewählt werden, um zu erreichen, dass in der Querrichtung möglichst nur ein Partikel der biologischen oder geologischen Probe gleichzeitig illuminiert wird. Die von der Probe reflektierten oder transmittierten Wellen werden durch das dispersive Element in unterschiedliche räumliche Richtungen abgelenkt. Die Nachweiselemente des Detektors sind zweidimensional angeordnet, wobei eine erste Richtung der Längsrichtung des beleuchteten Bereichs der Probe entspricht und die zweite Richtung der Dispersion des dispersiven Elements. Das erfindungsgemäße Spektrometer entspricht somit einer Nebeneinanderanordnung einer beliebig hohen Anzahl von einfachen Spektrometern entlang des beleuchteten Bereichs der Probe, sodass von dem untersuchten Bereich der Probe gleichzeitig eine hohe Anzahl an Spektren gewonnen werden können. Die einzelnen Spektren repräsentieren jeweils relativ kleine Bereiche der Probe, die kleiner als die Partikelgröße der Probe sein können, was die Messgenauigkeit des Spektrometers verbessert und für die gesamte Probe repräsentative Ergebnisse gewährleistet. Zudem besteht die Möglichkeit, nicht-repräsentative Spektren (Ausreißer) von der Auswertung auszunehmen.

Die Quelle kann die Probe mit breitbandigem Licht beaufschlagen, dessen Spektrum im fern- und/oder nahinfraroten und/oder sichtbaren Wellenlängenbereich liegen kann (vgl. EP 1 053 671 A1), um mittels einer Auswertungseinrichtung und einer vorhandenen Kalibrierung anhand der Spektren gewisse Inhaltsstoffe der Probe zu evaluieren. Bei einer anderen Ausführungsform ist die Quelle monochromatisch und ein für die Wellenlänge der Quelle (fast) undurchlässiger Filter kann zwischen die Probe und den Detektor einfügbar sein. Bei dieser Ausführungsform handelt es sich insbesondere um ein Raman-Spektrometer, bei dem es ebenfalls möglich ist, mittels einer Auswertungseinrichtung und einer vorhandenen Kalibrierung den Gehalt der Probe an bestimmten Inhaltsstoffen zu bestimmen. Es ist auch möglich, dass die Wellenlänge der Quelle unter mehreren Wellenlängen umschaltbar, insbesondere um zwischen einer Erfassung einer Stokes-Streuung und einer Anti-Stokes-Streuung zu wechseln. Auch kann der Filter, bei dem es sich um einen schmalbandigen oder einen Kantenfilter handeln kann, wechselbar sein. Der Detektor kann mehrere Ordnungen der dispergierten Wellen aufnehmen.

Weiterhin kann der Detektor mit einer Auswertungseinheit verbunden sein, deren Gehäuse räumlich von einem die Quelle, das Übertragungsmittel, das dispersive Element und den Detektor enthaltenden Gehäuse beabstandet und mit letzterem durch eine sich bei Überlast trennende mechanische und/oder elektrische Verbindung verbunden ist.

Die Spektrometeranordnung lässt sich stationär oder mobil einsetzen, um eine stationäre Probe zu untersuchen, oder die Probe wird an der Spektrometeranordnung (oder umgekehrt) vorbeigeführt, z.B. auf einem landwirtschaftlichen Feld, um Bodeneigenschaften und insbesondere Inhaltsstoffe des Bodens anhand der ermittelten Spektren zu evaluieren. Sie lässt sich auch zur Untersuchung der Inhaltsstoffe (z.B. Kalium oder Phosphor) von anderen, gasförmigen, festen oder flüssigen Proben (z.B. Gülle, Flüssigdünger, Spritzmitteln, Pflanzen oder Erntegut) einsetzen, sei es als Handgerät oder angebracht an einer Maschine zum Transportieren und/oder Ausbringen der Flüssigkeit oder an einer Erntemaschine. Die ermittelten Inhaltsstoffe und/oder daraus abgeleitete Daten der Probe können durch eine Rechnereinrichtung georeferenziert kartiert und/oder zur Ansteuerung einer landwirtschaftlichen Maschine verwendet werden, z.B. um die Ausbringung von Düngemitteln, Spritzmitteln, Saatgut, Siliermittel etc. zu kontrollieren.

### Ausführungsbeispiel

In den Zeichnungen ist ein nachfolgend näher beschriebenes Ausführungsbeispiel der Erfindung dargestellt. Es zeigt:
- Fig. 1: eine Draufsicht auf einen Ackerschlepper mit einer daran angebrachten Sämaschine,
- Fig. 2: eine seitliche Ansicht einer mit einer Spektrometereinrichtung ausgestatteten Reiheneinheit der Sämaschine,
- Fig. 3: eine schematische Seitenansicht der Spektrometereinrichtung,
- Fig. 4: eine Draufsicht auf die Spektrometereinrichtung,
- Fig. 5: ein Beispiel für von der Spektrometereinrichtung aufgenommene Spektren bei unterschiedlichen Anregungswellenlängen,
- Fig. 6: ein Schema für unterschiedliche Beugungsordnungen des dispersiven Elements, und
- Fig. 7: eine schematische, seitliche Ansicht des Gehäuses des Spektrometers mit einem weiteren Gehäuse, in dem eine Auswertungseinheit untergebracht ist.

### Landwirtschaftliche Maschine

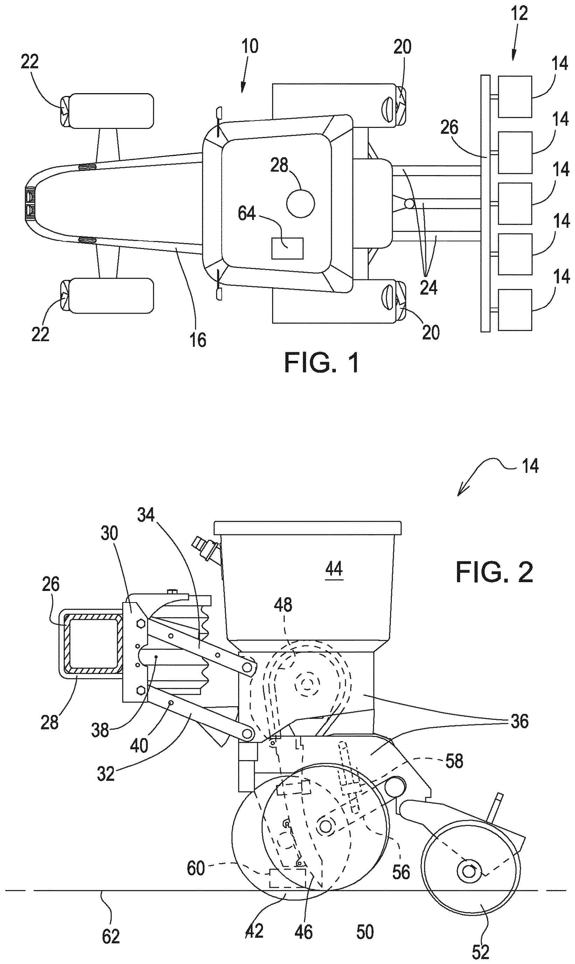

Die Figur 1 zeigt eine Draufsicht auf eine Kombination aus einem Ackerschlepper 10 und einer daran befestigten Sämaschine 12 mit einer Anzahl über die Breite des Geräts 12 verteilter Reiheneinheiten 14 zum Einbringen von Saatgut in den Boden, obwohl auch beliebige andere Typen von Sämaschinen 12 verwendet werden können. Die Reiheneinheiten 14 können auf einer Linie liegen, wie gezeigt, oder in Vorwärtsrichtung zueinander versetzt befestigt sein. Der Ackerschlepper 10 umfasst ein Fahrgestell 16 mit hinteren, antreibbaren Rädern 20 und vorderen, lenkbaren Rädern 22. An einer am Fahrgestell 16 befestigten Dreipunktanbauvorrichtung 24 mit Ober- und Unterlenkern ist ein sich horizontal und quer zur Vorwärtsrichtung des Ackerschleppers 10, die in der Figur 1 von rechts nach links verläuft, erstreckender Werkzeugträger 26 angebracht. Der Werkzeugträger 26 haltert die Reiheneinheiten 14 der Sämaschine 12. Die Sämaschine 12 könnte auch auf eine beliebige andere Art am Fahrzeug 12 befestigt sein, beispielsweise auf einem Fahrgestell ruhen und über eine Deichsel gezogen werden. Auch könnte die Sämaschine 12 als pneumatische Sämaschine ausgeführt sein. Eine auf dem Empfang von Satellitensignalen (GPS, Galileo und/oder Glonass und ggf. lokaler Korrektursignal) beruhende Positionsbestimmungseinrichtung 28 ist am Dach des Ackerschleppers 10 befestigt.

Die Figur 2 zeigt eine Reiheneinheit 14 der Sämaschine 12. Die Befestigung der Reiheneinheit 14 am Werkzeugträger 26 erfolgt über U-förmige Halterungen 28, die mit einer Konsole 30 verbunden sind, die sich vertikal an der Rückseite des Werkzeugträgers 26 erstreckt und an der übereinander zwei Lenker 32, 34 angelenkt sind, die außerdem jeweils an einem Gestell 36 der Reiheneinheit 14 angelenkt sind. Die Lenker 32, 34 bilden mit der Konsole 30 und dem Gestell 36 ein verstellbares Parallelogramm, das die Höhe des Gestells 36 über dem Boden definiert. Ein als Vorspannmittel dienender pneumatischer Aktor 38, der in der dargestellten Ausführungsform als pneumatischer Balgzylinder ausgeführt ist, greift einerseits oben an der Konsole 30 und andererseits unten am unteren Lenker 32 (am Punkt 40) an und definiert die Position des Gestells 36 und die Andruckkraft, mit der ein am Gestell 36 abgestützte6 Furchenöffner 42 mit dem Erdboden zusammenwirkt.

Das Gestell 36 trägt in an sich bekannter Weise einen Saatgutbehälter 44, ein Saatgutrohr 46 sowie eine (insbesondere pneumatische, mit von einem nicht gezeigten Gebläse bereitgestellten Unterdruck arbeitende) Zumesseinrichtung 48, die nach und nach einzelne Körner des Saatguts aus dem Saatgutbehälter 44 in das Saatgutrohr 46 abgibt, welches es in eine Furche ablegt, die durch den Furchenöffner 42 erzeugt wird, dessen Arbeitstiefe durch ein Tiefeneinstellrad 50 vorgegeben wird. Die Furche wird durch ein Schließrad 52 geschlossen. Die vertikale Position einer das Tiefeneinstellrad 50 halternden Schwinge 56 und somit die Tiefe der Furche, in welcher das Saatgut abgelegt wird, wird durch einen Aktor 58 definiert. Ein nicht gezeigter Rückkopplungssensor kann die aktuelle Sätiefe erfassen.

Am unteren Ende des Gestells 36 ist vor dem Saatgutrohr 46 eine Spektrometeranordnung 60 angebracht, die mit dem Erdboden 62 und insbesondere einer vom Furchenöffner 42 erzeugten Furche zusammenwirkt. Die Spektrometeranordnung 60 ermittelt auf optische Weise Eigenschaften des Erdbodens 62, z.B. hinsichtlich dessen Inhaltsstoffen, und übermittelt ihre Messwerte an eine Rechnereinrichtung 64 des Ackerschleppers 10. Dort wird sie, unter Verwendung der Signale von der Positionsbestimmungseinrichtung 28, zur Planung nachfolgender Arbeitsvorgänge georeferenziert abgespeichert und/oder dient zur Ansteuerung eines Aktors, z.B. des Aktors 58 zur Vorgabe der Sätiefe. Die dargestellte Anbringung der Spektrometeranordnung 60 ist nur ein Ausführungsbeispiel. Die Spektrometeranordnung könnte an beliebigen anderen (landwirtschaftlichen) Maschinen angebracht werden und von diesen über den Boden 62 bewegt werden, um dessen Eigenschaften zu bestimmen. Auch könnte sie stationär oder mobil eingesetzt werden, um Bodenproben zu untersuchen, oder sie kann zur Analyse der Inhaltsstoffe beliebiger anderer Materialen verwendet werden (z.B. von Gülle oder Fest- oder Flüssig-Dünger oder zu spritzenden oder düngenden Pflanzen oder zu erntendem oder geerntetem Erntegut). Die gewonnenen Daten hinsichtlich der Inhaltsstoffe können zur Ansteuerung von Aktoren z.B. zur Ausbringung von Hilfsstoffen wie Dünger oder Spritzmittel dienen oder georeferenziert abgespeichert werden, um sie zu präzisionslandwirtschaftlichen Zwecken zu nutzen.

### Spektrometeranordnung

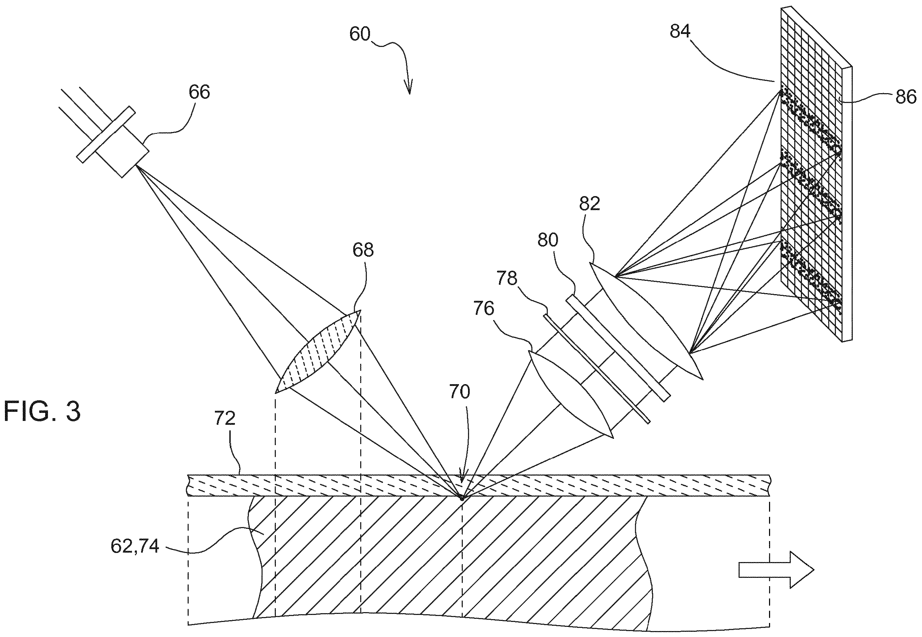

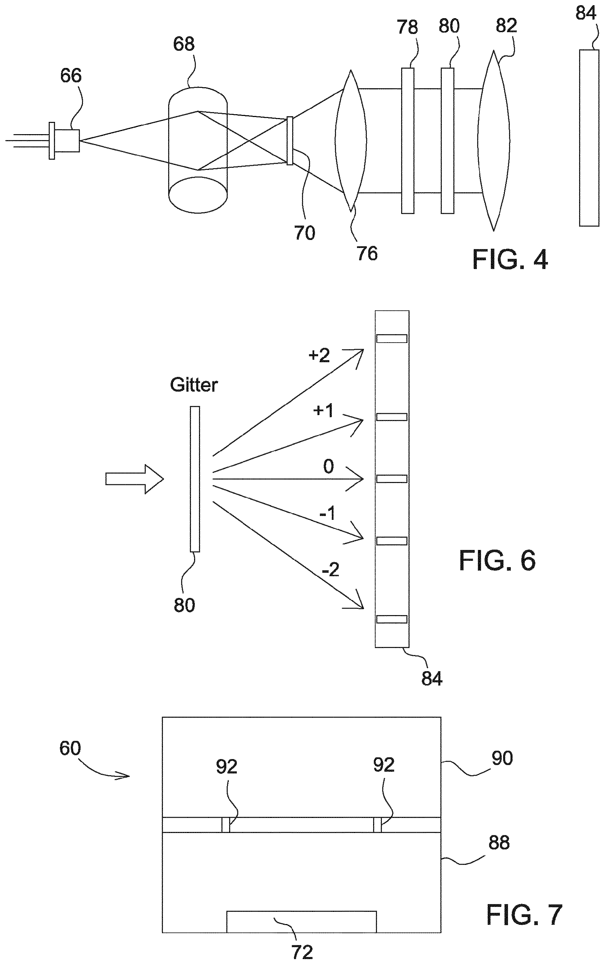

Es wird nun auf die Figuren 3 und 4 verwiesen, in denen der Aufbau der Spektrometeranordnung 60 detaillierter dargestellt ist. Die in der dargestellten Ausführungsform als Raman-Spektrometer ausgestaltete Spektrometeranordnung 66 umfasst eine Quelle 66 für elektromagnetische Wellen, bei der es sich um eine Laserdiode handeln kann, Übertragungsmittel 68 z.B. in Form einer Zylinderlinse, die dazu dienen, die elektromagnetischen Wellen von der Quelle 66 durch ein für die Wellen durchlässiges Fenster 72 hindurch zu einer an der Außenseite des Fensters 72 (und somit außerhalb der Spektrometeranordnung 60) befindlichen beleuchteten Fläche 70 auf einer zu untersuchenden Probe 74 zu lenken und auf die Fläche 70 zu fokussieren. Die elektromagnetischen Wellen von der Quelle 66 können im sichtbaren oder (nah-) infraroten Bereich liegen. Bei der Probe 74 kann es sich insbesondere um den Erdboden 62 handeln, wie im Ausführungsbeispiel der Figur 2 gezeigt, oder eine beliebige andere, biologische oder geologische Probe 74. Die Übertragungsmittel 68 sind derart ausgeformt, dass die beleuchtete Fläche 70 strichförmig ist, wie in der Figur 4 dargestellt ist. Es können zusätzliche, in den Figuren 3 und 4 nicht gezeigte Mittel (z.B. Linsen, Prismen, bewegliche Elemente o. dgl.) verwendet werden, um von der nahezu punktförmigen Quelle 66 ausgehenden, näherungsweise parallel verlaufenden Wellen strichförmig aufzufächern.

Das Fenster 72 kann aus Saphirglas sein, um die Abrasion durch die Probe 74 gering zu halten. Anders als in der Figur 3 gezeigt, kann sich die beleuchtete Fläche 70 in einem gewissen Abstand von einigen mm oder cm vom Fenster 72 befinden.

Weiterhin umfasst die Spektrometeranordnung 60 zur Analyse von der Probe 74 reflektierten, durch das Fenster 72 wieder in die Spektrometeranordnung 60 eintretenden, elektromagnetischen Wellen ein dispersives Element 80 in Form eines Gitters (oder Prismas) und einen Detektor 84 mit Nachweiselementen 86, die auf sie auftreffende elektromagnetische Wellen in elektrische Signale umsetzen. Das Gitter kann als Spiegelgitter oder holographisches Gitter ausgeführt sein. Der Detektor 84 kann als CCD- oder Photodiodenmatrix ausgeführt sein. Zwischen der Probe 74 und dem dispersiven Element 80 kann eine Linse 76 angeordnet sein, welche die von der Probe 74 einlaufenden, divergierenden Wellen zu parallel verlaufenden Wellen formt, und zwischen dem dispersiven Element 80 und dem Detektor 84 kann eine (insbesondere das dispersive Element 80 auf den Detektor 84 abbildende) Fokussieroptik 82 angebracht sein. Ein Filter 78 kann vor oder hinter dem dispersiven Element 80 angeordnet werden, um die bei der Auswertung der Spektren störende Wellenlänge der monochromatischen Quelle 66 auszufiltern bzw. zu dämpfen. Bei der Linse 76 und der Fokussieroptik 82 kann es sich um sphärische oder asphärische Linsen oder komplexere Mehrlinsenaufbauten oder andere optische Elemente handeln.

Die Längsachse der als Übertragungsmittel 68 dienenden Zylinderlinse erstreckt sich quer zur Zeichenebene der Figur 3 und in der Zeichenebene der Figur 4 vertikal und somit parallel zur Längsrichtung der beleuchteten Fläche 70. Die Gitterspalte des dispersiven Elements 80 erstrecken sich ebenfalls quer zur Ebene der Figur 3. Die optischen Achsen der Linse 76 und der Fokussieroptik 82 liegen in der Ebene der Figur 3.

### Wirkungsweise

Die Probe 74 wird nach alledem innerhalb einer strichförmigen Fläche 70 mit monochromatischen Wellen (Licht) illuminiert. Die von der Probe 74 reflektierten Wellen gelangen zum dispersiven Element 80 und werden durch dieses in wellenlängenabhängigen Richtungen abgelenkt, d.h. in der Zeichenebene der Figur 3 in unterschiedliche Winkel dispergiert. Dadurch empfangen die Nachweiselemente 86 des Detektors 84 entlang der Zeichenebene der Figur 3 (die im Rahmen der vorliegenden Offenbarung als zweite Richtung bezeichnet wird) unterschiedliche Intensitäten, die von der jeweiligen Wellenlänge abhängen. Es wird in den übereinander liegenden Nachweiselementen 86 der Figur 3 demnach jeweils ein Spektrum erfasst. Aufgrund der strichförmig beleuchteten Fläche 74 entstehen in der quer zur Zeichenebene der Figur 3 liegenden Richtung (die im Rahmen der vorliegenden Offenbarung als erste Richtung bezeichnet wird) auf dem Detektor 84 mehrere, voneinander unabhängige Spektren, die jeweils unterschiedliche Stellen der Probe 74 repräsentieren.

Die Spektrometeranordnung 60 erfasst demnach gleichzeitig die Spektren mehrerer nebeneinander liegender, relativ kleiner Bereiche der Probe 74. Dadurch wird eine hinreichende Intensität des Lichts ermöglicht und zudem ist sichergestellt, dass bei der Auswertung der Signale der Nachweiselemente durch eine in den Figuren nicht dargestellte Auswertungseinheit, die räumlich mit der Spektrometeranordnung 60 in einem Gehäuse integriert oder davon getrennt sein kann (z.B. in die Rechnereinrichtung 64 integriert oder auf einem separaten Rechner, der durch Datenfernübertragung oder eine transportable Speicherkarte mit den zwischengespeicherten Signalen der Nachweiselemente 86 beaufschlagbar sein kann), nicht repräsentative Bereiche der Probe 74, die sich durch entlang der zweiten Richtung durch die Nachweiselemente 86 aufgenommene Spektren erkennen lassen, die sich signifikant (um mehr als einen Schwellenwert) von den Spektren unterscheiden, die in den übrigen Bereichen der Probe 74 erzeugt und von den anderen Nachweiselementen 86 des Detektors 84 entlang der zweiten Richtung aufgenommen werden, bei der Evaluierung der Inhaltsstoffe ggf. ausgesondert werden können, oder ihre verfälschende Wirkung auf das Ergebnis wird zumindest durch Mittelung gemildert. Es kann eine Mittelung der einzelnen Spektren oder der berechneten Inhaltsstoffe erfolgen, die anhand der einzelnen Spektren und einer Kalibrierung ermittelt werden.

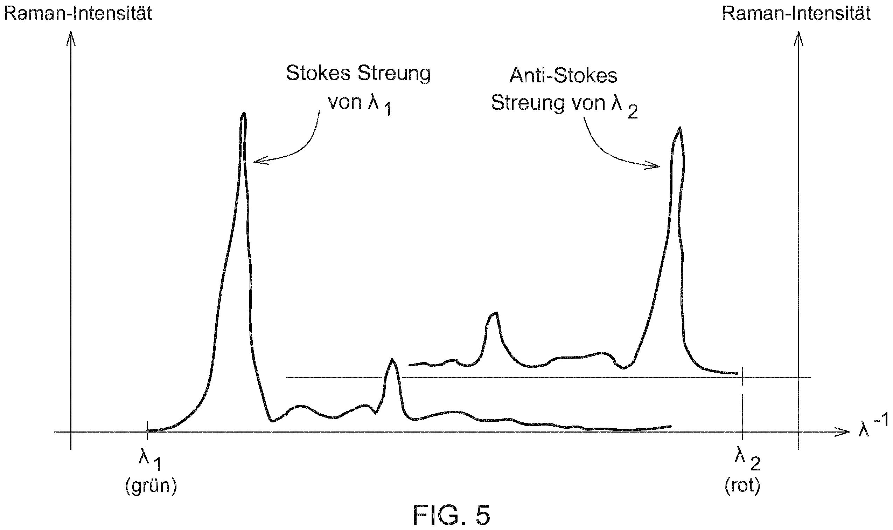

Die Figur 5 zeigt beispielhaft ein vom Detektor 84 durch dessen Nachweiselemente 86 entlang der zweiten Richtung aufgenommenes Spektrum (die X-Achse ist proportional zur Wellenzahl oder 1/λ), bei welchem die Probe 74 von der Quelle 66 mit einer monochromatischen (hier: grünen) Wellenlänge λ₁ beaufschlagt wird. Die Anregungswellenlänge λ₁ der Quelle 66 wird durch den Filter 78 ausgefiltert oder gedämpft und das Spektrum umfasst im Wesentlichen die von der Wellenlänge λ₁ angeregte, so genannte Stokes-Streuung, die dadurch entsteht, dass Vibrations- und Rotationsmoden von Oberflächenmolekülen der Probe 74 angeregt werden, weshalb die von der Probe 74 reflektierten Wellen langwelliger sind als die Wellenlänge λ₁ und infolge der Energieabgabe an die Anregungsmoden der Moleküle weniger Energie enthalten.

Die Figur 5 zeigt zudem ein zweites Spektrum, bei welchem die Probe 74 von der Quelle 66 mit einer monochromatischen (hier: roten) Wellenlänge λ₂ beaufschlagt wird. Die Anregungswellenlänge λ₂ der Quelle 66 wird durch den (nunmehr anderen) Filter 78 ausgefiltert und das Spektrum umfasst im Wesentlichen die von der Wellenlänge λ₂ angeregte, so genannte Anti-Stokes-Streuung, die dadurch entsteht, dass die von der Probe reflektierten Wellen zusätzliche Energie von Vibrations- und Rotationsmoden von Oberflächenmolekülen der Probe 74 aufnehmen, weshalb die von der Probe 74 reflektierten Wellen kurzwelliger sind als die Wellenlänge λ₂ und infolge der Energieaufnahme aus den Anregungsmoden der Moleküle mehr Energie enthalten.

Es wäre auch möglich, mit einer einzigen Wellenlänge, die zwischen λ₁ und λ₂ liegt, sowohl die Anti-Stokes-Streuung als auch die Stokes-Streuung anzuregen und zu analysieren.

### Weitere Ausführunasformen

Es ist demnach denkbar, die von der Quelle 66 abgegebene Wellenlänge umschaltbar zu machen, z.B. um je nach Anwendungsfall die Stokes-Streuung oder die Anti-Stokes-Streuung auswerten zu können oder normalerweise bei einer ersten, kurzwelligen Wellenlänge arbeiten und im Falle von Auftreten von Fluoreszenz auf eine längere Wellenlänge umschalten zu können, die keine Fluoreszenz erzeugt. Dazu könnte die Quelle 66 zwei oder mehr unterschiedliche Erzeuger für elektromagnetische Wellen umfassen, zwischen denen elektrisch und/oder durch mechanische Bewegung umgeschaltet wird, sodass unabhängig von der Wellenlänge stets dieselbe Fläche 70 beaufschlagt wird. Der Filter 78 wäre dann ebenfalls zu wechseln, wozu ein entsprechender Aktor vorgesehen werden kann, oder es wird ein einziger Filter 78 verwendet, der die beiden Wellenlängen der Quellen 66 ausfiltert oder dämpft oder es werden zwei Filter 78 hintereinander angeordnet, von denen jeweils einer eine Wellenlänge einer der Quellen 66 ausfiltert oder dämpft. Es können jeweils schmalbandige oder Kantenfilter verwendet werden. Der Filter 78, welcher die Intensität der Anregungswellenlänge um mehrere Zehnerpotenzen reduziert, um eine Referenzierung der Wellenlänge der Quelle 66 und ihrer Intensität zur Auswertung der Spektren zu ermöglichen, dämpft im Bereich der jeweiligen Anregungswellenlängen. Die zu untersuchenden Wellenlängen werden durch den oder die Filter 78 jedoch durchgelassen.

Kürzere Anregungswellenlängen haben in der Raman-Spektroskopie Vorteile, können aber z.B. durch das Auftreten von Fluoreszenz nicht immer genutzt werden. Längere Anregungswellenlängen haben energetische Nachteile, sind aber in gewissen Anwendungsfällen die einzige Lösung, um Raman-Spektroskopie betreiben zu können. Die Anregungswellenlängen der Quellen 66 können insbesondere an den Anfang und an das Ende des von der Spektrometeranordnung 60 verarbeitbaren Spektralbereiches gelegt werden. Wenn die Quelle 66 mit der kürzeren Wellenlänge arbeitet, wird die Stokes-Streuung genutzt, und wenn die Quelle 66 mit der längeren Wellenlänge arbeitet, wird die Anti-Stokes-Streuung genutzt. Die Quellen 66 werden demnach alternierend und nicht gleichzeitig aktiviert.

Der vom Spektrometer optimal ausgenutzte Spektralbereich liege beispielsweise zwischen ca. 650 nm und ca. 900 nm. Eine Quelle 66 in Form eines Halbleiterlasers von 660 nm Wellenlänge könnte zur Anregung des Ramanspektrums dienen, das die Stokes-Streuung benutzt und für die interessierenden Substanzen im Bereich 665 nm bis 850 nm liegt. Die zweite Quelle 66 in Form eines zweiten Anregungslasers könnte bei ca. 900 nm liegen. Das Ramanspektrum aus der Anti-Stokes-Streuung würde dann den Bereich 895 nm bis 700 nm ausnutzen. In der Stokes-Streuung und in der Anti-Stokes-Streuung befinden sich ähnliche aber nicht identische Informationen über die Probe. Durch den Wechsel der Anregungswellenlängen sind spezielle Informationen zu gewinnen. Die Quellen 66 können alternierend betrieben werden oder es erfolgt (automatisiert, abhängig vom detektierten Spektrum) ein Wechsel der Anregungswellenlängen, z.B. um ggf. auftretende Fluoreszenz zu verhindern. Die Filter für die Dämpfung der Laserwellenlänge können bei dieser Ausführungsform Kantenfilter sein und müssen nicht (wie bei der im folgenden Absatz beschriebenen Nutzung von mehreren Ordnungen) sehr schmalbandige Filter sein. Außer der bereits erwähnten Vermeidung von Fluoreszenz kann man durch geschickte Wahl der Anregungswellenlänge erreichen, dass das Ramanspektrum im empfindlichsten Spektralbereich des Detektionssystems liegt.

Weiterhin wäre denkbar, dass das dispersive Element 80 und der Detektor 84 wie in der Figur 6 dargestellt ausgeführt sein können, d.h. der Detektor 84 erfasst nicht nur, wie im Stand der Technik üblich, die nullte und erste Beugungsordnung, sondern auch die zweite Beugungsordnung oder ggf. noch höhere Beugungsordnungen. Mit jeder Ordnung kann man eine spezielle Wellenlänge der Quelle 66 optimal nutzen. Zudem wird durch die Auswertung der höheren Ordnungen die gesamte, vom Detektor 84 bereitgestellte Information über die Probe 74 evaluiert.

Schließlich ist in der Figur 7 dargestellt, dass die Spektrometeranordnung 60 in einem Gehäuse mit einem ersten Teil 90 und einem zweiten Teil 88 angebracht sein kann. Der zweite, untere Teil 88 enthält die (in den Figuren 3 und 4 gezeigten) optischen Elemente der Spektrometeranordnung 60, einschließlich der Quelle 66 und dem Detektor 84 sowie einen diesem zugeordneten Analog-Digital-Wandler zur Digitalisierung der Signale der Nachweiselemente 86 und einen Speicher mit Identifikationsdaten und/oder spezifischen Parametern der Spektrometeranordnung 60, die z.B. durch die oben beschriebene Auswertungseinheit zur Auswertung der Signale der Nachweiselemente 86 genutzt werden können, während der erste, obere Teil 90 die zur Auswertung der Signale des Detektors 84 benötigten Auswertungseinheit, einen Speicher, Stromversorgung und eine Schnittstelle zur Rechnereinrichtung 64 umfasst. Der erste Teil 90 wird fest an der landwirtschaftlichen Maschine (z.B. wie in Figur 2 am Gestell 36 der Reiheneinheit 14 der Sämaschine 12) montiert und ist durch eine sich bei Überlast trennende Verbindung in Form einer Sollbruchstelle, die z.B. durch Abscherstifte 92 realisiert werden kann, mit dem zweiten Teil 88 verbunden. Falls demnach der zweite Teil 88 bei unerwartetem Bodenkontakt beschädigt wird, reißt die Sollbruchstelle ab und der erste Teil 90 bleibt intakt. In diesem Fall trennt sich auch eine elektrische Steckverbindung zwischen dem ersten Teil 90 und dem zweiten Teil 88, die in der Figur 7 nicht gezeigt ist.

Man könnte die beiden Teile 90 und 88 auch räumlich weiter voneinander trennen. So könnte der erste Teil 90 an Bord des Ackerschleppers 10 oder weiter oben an der Sämaschine 12 angebracht werden und durch ein elektrisches Verbindungskabel mit dem zweiten Teil 88 gekoppelt werden, der sich bei einer derartigen Ausführungsform direkt oder indirekt am Gestell 36 der Sämaschine 12 abstützen würde und durch eine sich bei Überlast trennende Verbindung in Form einer Sollbruchstelle mit dem Gestell 36 verbunden wäre. In diesem Fall würde sich auch eine Steckverbindung des Verbindungskabels zwischen den beiden Teilen 90 und 88 lösen. Somit wäre auch bei dieser Ausführungsform gewährleistet, dass maximal der zweite Teil 88 zu ersetzen wäre.

Es sei noch angemerkt, dass das zweiteilige Gehäuse der Figur 7 auch mit Spektrometeranordnungen 60 verwendet werden kann, deren Detektor 84 nur eine einzige Zeile an Nachweiselementen 86 umfasst. Analoges gilt für die erwähnte, umschaltbare Wellenlänge und die Nutzung mehrerer Ordnungen.

Die Ein- und Ausfallswinkel der Wellen auf die Probe 74 müssen nicht, wie in der Figur 3 gezeigt, jeweils etwa 45° betragen, sondern können davon abweichen und auch unsymmetrisch sein.

Die Breite der Fläche 70 in der horizontalen Zeichenebene der Figur 3 kann konstant sein oder durch Verstellen der Übertragungsmittel 68 variabel sein, z.B. zur Anpassung an die Partikelgröße der Probe 74. Es wäre aber auch denkbar, einen festen Spalt zwischen der Quelle 66 und der Probe 74 einzusetzen, der die Übertragungsmittel 68 ersetzt oder ergänzt. Hat der Spalt eine Taille oder ist er als Dreieck oder stufig ausgeführt, kann die Breite der Fläche 70 in der horizontalen Zeichenebene der Figur 3 über die Länge der Fläche 70 variieren, um die Auflösung und Empfindlichkeit der Spektrometeranordnung 60 variieren oder optimieren zu können. Die Änderung der Breite des Eintrittsspaltes kann zeitlich nacheinander erfolgen oder jedes Spektrum oder Gruppen von Spektren haben simultan unterschiedliche Breiten.

## Patentansprüche

1. Landwirtschaftliche Maschine mit einer Spektrometeranordnung (60) zur Untersuchung einer Probe (74), die Spektrometeranordnung umfassend
eine Quelle (66) für elektromagnetische Wellen,
Übertragungsmittel (68) zum Übertragen der Wellen von der Quelle (66) zur Probe (74),
ein dispersives Element (80), das mit von der Probe (74) einlaufenden Wellen beaufschlagbar ist und geeignet ist, die einlaufenden Wellen wellenlängenabhängig in unterschiedliche Richtungen abzulenken, und
einen Detektor (84) mit mehreren, zweidimensional angeordneten Nachweiselementen (86) zur Umsetzung der vom dispersiven Element (80) einlaufenden Wellen in elektrische Signale,
wobei die Übertragungsmittel (68) ausgelegt sind, die Probe (74) strichförmig mit den elektromagnetischen Wellen zu beleuchten
und die Spektrometeranordnung (60) derart konfiguriert ist, dass die von unterschiedlichen Orten der strichförmig mit elektromagnetischen Wellen beaufschlagten Probe (74) ausgehenden elektromagnetischen Wellen entlang einer ersten Richtung an unterschiedlichen Nachweiselementen (86) des Detektors (84) und aufgrund der durch das dispersive Element (80) bewirkten, wellenlängenabhängigen Ablenkung entlang einer zweiten Richtung an unterschiedlichen Nachweiselementen (86) des Detektors (84) einlaufen; wobei die Spektrometeranordnung (60) in einer zur Untersuchung von an der Spektrometeranordnung (60) vorbeilaufenden Proben (74) geeigneten Position an der Maschine angebracht und mit einer Rechnereinrichtung (64) zur georeferenzierten Aufzeichnung von Ausgangssignalen der Spektrometeranordnung (60) und/oder zur darauf basierten Ansteuerung eines Aktors (58) programmiert ist.

2. Landwirtschaftliche Maschine nach Anspruch 1, wobei die Quelle (66) punktförmig ist und die Übertragungsmittel (68) eingerichtet sind, die Quelle (66) auf die strichförmig beleuchtete Fläche (70) der zu untersuchenden Probe (74) abzubilden.

3. Landwirtschaftliche Maschine nach einem der vorhergehenden Ansprüche, wobei das dispersive Element (80) ein Gitter umfasst.

4. Landwirtschaftliche Maschine nach einem der vorhergehenden Ansprüche, wobei die Quelle (66) monochromatisch ist und ein für die Wellenlänge der Quelle (66) zumindest näherungsweise undurchlässiger Filter (78) zwischen die Probe (74) und den Detektor (84) einfügbar oder eingefügt ist.

5. Landwirtschaftliche Maschine nach Anspruch 4, wobei die Wellenlänge der Quelle (66) unter mehreren Wellenlängen umschaltbar und/oder der Filter (78) wechselbar ist.

6. Landwirtschaftliche Maschine nach Anspruch 4 oder 5, wobei der Filter (78) ein schmalbandiger Filter oder ein Kantenfilter ist.

7. Landwirtschaftliche Maschine nach einem der Ansprüche 1 bis 6, wobei das dispersive Element (80) und der Detektor (84) derart dimensioniert sind, dass die Nachweiselemente (86) des Detektors (84) wenigstens eine über eine erste Ordnung hinausgehende Ordnung der dispergierten Wellen aufnehmen.

8. Landwirtschaftliche Maschine nach einem der Ansprüche 1 bis 7, wobei der Detektor (84) mit einer Auswertungseinheit verbunden ist, die programmiert ist, anhand der Signale des Detektors (84) und einer Kalibrierung Inhaltsstoffe der Probe (74) zu berechnen und entlang der zweiten Richtung durch die Nachweiselemente (86) aufgenommene Spektren, die sich um mehr als einen Schwellenwert von den Spektren unterscheiden, die von den anderen Nachweiselementen (86) des Detektors (84) entlang der zweiten Richtung aufgenommen werden, bei der Bestimmung der Inhaltsstoffe der Probe (74) nicht zu berücksichtigen.

9. Landwirtschaftliche Maschine nach einem der vorhergehenden Ansprüche, wobei der Detektor (84) mit einer Auswertungseinheit verbunden ist, die in einem ersten Gehäuse (90) angeordnet ist, welches räumlich von einem die Quelle (66), das Übertragungsmittel, das dispersive Element und den Detektor (84) enthaltenden, zweiten Gehäuse (88) getrennt und durch eine sich bei Überlast trennende, mechanische und/oder elektrische Verbindung mit dem zweiten Gehäuse (88) verbunden ist.

## Claims

1. Agricultural machine with a spectrometer assembly (60) for examining a sample (74), the spectrometer assembly comprising:
a source (66) for electromagnetic waves, transmission means (68) for transmitting the waves from the source (66) to the sample (74),
a dispersive element (80) to which waves coming from the sample (74) can be applied, which dispersive element (80) is suitable for deflecting the incoming waves in different directions as a function of the wavelength, and
a detector (84) having a plurality of detection elements (86) which are arranged in a two-dimensional fashion and have the purpose of converting the waves which are received from the dispersive element (80) into electrical signals,
wherein the transmission means (68) are configured to illuminate the sample (74) in lines with the electromagnetic waves,
and the spectrometer assembly (60) is configured in such a way that the electromagnetic waves which originate from different locations of the sample (74), to which electromagnetic waves are applied in lines, arrive from a first direction at different detection elements (86) of the detector (84) and from a second direction at different detection elements (86) of the detector (84) owing to the wavelength-dependent deflection which is brought about by the dispersive element (80); wherein the spectrometer assembly (60) is mounted at a position on the machine which is suitable for examining samples (74) which run past the spectrometer assembly (60) and is programmed with a computer device (64) to perform georeferenced recording of output signals of the spectrometer assembly (60) and/or to carry out actuation of an actuator (58) on the basis thereof.

2. Agricultural machine according to Claim 1, wherein the source (66) is punctiform and the transmission means (68) are configured to map the source (66) onto that face (70) of the sample (74) to be examined which is illuminated in lines.

3. Agricultural machine according to one of the preceding claims, wherein the dispersive element (80) comprises a grid.

4. Agricultural machine according to one of the preceding claims, wherein the source (66) is monochromatic, and a filter (78) which is at least approximately impermeable to the wavelength of the source (66) can be inserted or is inserted between the sample (74) and the detector (84).

5. Agricultural machine according to Claim 4, wherein the wavelength of the source (66) can be switched between a plurality of wavelengths and/or the filter (78) can be replaced.

6. Agricultural machine according to Claim 4 or 5, wherein the filter (78) is a narrow band filter or an edge filter.

7. Agricultural machine according to one Claims 1 to 6, wherein the dispersive element (80) and the detector (84) are dimensioned in such a way that the detection elements (86) of the detector (84) receive at least one order, greater than a first order, of the dispersed waves.

8. Agricultural machine according to one Claims 1 to 7, wherein the detector (84) is connected to an evaluation unit which is programmed to calculate constituents of the sample (74) on the basis of the signals of the detector (84) and a calibration process, and not to take into account in the determination of the constituents of the sample (74) spectra which are picked up in the second direction by the detection elements (86) and which differ by more than a threshold value from the spectra which are picked up by the other detection elements (86) of the detector (84), in the second direction.

9. Agricultural machine according to one of the preceding claims, wherein the detector (84) is connected to an evaluation unit which is arranged in a first housing (90) which is spatially separate from a second housing (88) which contains the source (66), the transmission means, the dispersive element and the detector (84), and said housing (90) is connected to the second housing (88) by means of a mechanical and/or electrical connection which disconnects in the event of an overload.

## Revendications

1. Machine agricole comprenant un ensemble formant spectromètre (60) destiné à examiner un échantillon (74), l'ensemble formant spectromètre comprenant :
une source (66) d'ondes électromagnétiques,
des moyens de transmission (68) destinés à transmettre les ondes de la source (66) à l'échantillon (74),
un élément dispersif (80) qui est soumis à des ondes incidentes (74) provenant de l'échantillon et qui est approprié pour dévier les ondes incidentes dans différentes directions en fonction de la longueur d'onde, et
un détecteur (84) comprenant une pluralité d'éléments de détection (86) disposés en deux dimensions et destinés à convertir les ondes incidentes provenant de l'élément dispersif (80) en signaux électriques,
les moyens de transmission (68) étant conçus pour éclairer l'échantillon (74) sous forme linéaire avec les ondes électromagnétiques et l'ensemble formant spectromètre (60) étant conçu de manière à ce que les ondes électromagnétiques provenant de différents emplacements de l'échantillon (74) soumis sous forme linaire à des ondes électromagnétiques soient incidentes le long d'une première direction à différents éléments de détection (86) du détecteur (84) et, en raison de la déviation dépendante de la longueur d'onde et produite par l'élément dispersif (80), dans différents éléments de détection (86) du détecteur (84) le long d'une deuxième direction ; l'ensemble formant spectromètre (60) étant fixé à la machine dans une position appropriée pour examiner des échantillons (74) passant devant l'ensemble formant spectromètre (60) et étant programmé au moyen d'un dispositif informatique (64) pour enregistrer, de manière géo-référencée, des signaux de sortie de l'ensemble formant spectromètre (60) et/ou pour commander sur cette base un actionneur (58) .

2. Machine agricole selon la revendication 1, la source (66) étant ponctuelle et les moyens de transmission (68) étant adaptés pour reproduire la source (66) sur la surface (70), éclairée linéairement, de l'échantillon (74) à examiner.

3. Machine agricole selon l'une des revendications précédentes, l'élément dispersif (80) comprenant une grille.

4. Machine agricole selon l'une des revendications précédentes, la source (66) étant monochromatique et un filtre (78), au moins approximativement imperméable à la longueur d'onde de la source (66), étant ou pouvant être inséré entre l'échantillon (74) et le détecteur (84).

5. Machine agricole selon la revendication 4, la longueur d'onde de la source (66) étant commutable entre plusieurs longueurs d'onde et/ou le filtre (78) étant remplaçable.

6. Machine agricole selon la revendication 4 ou 5, le filtre (78) étant un filtre à bande étroite ou un filtre de bord.

7. Machine agricole selon l'une des revendications 1 à 6, l'élément dispersif (80) et le détecteur (84) étant dimensionnés de telle sorte que les éléments de détection (86) du détecteur (84) détectent au moins un ordre des ondes dispersées qui va au-delà d'un premier ordre.

8. Machine agricole selon l'une des revendications 1 à 7, le détecteur (84) étant relié à une unité d'évaluation qui est programmée pour calculer des constituants de l'échantillon (74) à partir des signaux du détecteur (84) et d'un étalonnage et pour ne pas prendre en compte, lors de la détermination des constituants de l'échantillon (74), des spectres qui sont détectés par les éléments de détection (86) le long de la deuxième direction et qui diffèrent de plus d'une valeur seuil des spectres qui sont détectés par les autres éléments de détection (86) du détecteur (84) le long de la deuxième direction.

9. Machine agricole selon l'une des revendications précédentes, le détecteur (84) étant relié à une unité d'évaluation qui est disposée dans un premier boîtier (90) qui est séparé spatialement d'un deuxième boîtier (88) contenant la source (66), le milieu de transmission, l'élément dispersif et le détecteur (84) et étant relié au deuxième boîtier (88) par une liaison mécanique et/ou électrique qui est interrompue en cas de surcharge.
